Europäisches Patentamt

European Patent Office

Office européen des brevets

(11) Publication number: **0 107 400**
**B1**

# EUROPEAN PATENT SPECIFICATION

(45) Date of publication of patent specification: 31.05.89

(21) Application number: 83305914.0

(22) Date of filing: 29.09.83

(51) Int. Cl.⁴: **C 12 N 15/00**, C 12 P 21/02, C 12 N 1/20 // C12R1/19

(54) Hybrid plasmid and process for producing glutathione using said plasmid.

(30) Priority: 29.09.82 JP 170727/82

(43) Date of publication of application:
02.05.84 Bulletin 84/18

(45) Publication of the grant of the patent:
31.05.89 Bulletin 89/22

(84) Designated Contracting States:
DE FR GB IT

(56) References cited:
EP-A-0 071 486

CHEMICAL ABSTRACTS, vol. 95, no. 6, September 1981, no. 95457c, Columbus, Ohio (US); M.KOUSAKU et al.: "Glutathione production coupled with an ATP regeneration system", p. 464

(73) Proprietor: Kimura, Akira
No. 80-2, Kamiwakamiya-cho Wakamiyadori rokujoagaru
Shimoko-ku Kyoto-shi Kyoto (JP)

(72) Inventor: Kimura, Akira
No.80-2,Kamiwakamiya-cho, Wakamiyadori rukujoagaru
Shimokyoto-ku, Kyoto-shi, Kyoto (JP)
Inventor: Murata, Kousaka
No. 3-35, Nishino hitsugawa-cho
Yamashina-ku Kyoto-shi Kyoto (JP)
Inventor: Miya, Toyofumi
No. 15-37, Gokasho shinkai
Uji-shi Kyoto (JP)
Inventor: Gushima, Hiroshi
No. 22-8, Asama-Dai 3-chome
Ageo-shi Saitama (JP)

(74) Representative: Geering, Keith Edwin et al
REDDIE & GROSE 16 Theobalds Road
London WC1X 8PL (GB)

The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## EP 0 107 400 B1

References cited:

CHEMICAL ABSTRACTS, vol. 93, no. 7, August 1980, no. 68651p, Columbus, Ohio (US); M.KOUSAKU et al.: "Continous production of glutathione using immobilized microbial cells containing ATP generating system", p. 732

CHEMICAL ABSTRACTS, vol. 92, no. 13, March 1980, no. 109142y, p. 512, Columbus, Ohio (US) AGRICULTURAL AND BIOLOGICAL CHEMISTRY, vol. 47, no. 6, June 1983, Tokyo (JP); K.MURATA et al.: "Cloning and amplification of a gene for glutathione synthetase in Escherichia coli B", pp. 1381-1383

APPLIED AND ENVIRONMENTAL MICROBIOLOGY, vol. 44, no. 6, December 1982, American Society for Microbiology (US); K.MURATA et al.: "Cloning of a gene responsible for the biosynthesis of flutathione in Escherichia coli B", pp. 1444-1448

## Description

The present invention relates to a hybrid plasmid having a gene for glutathione bio synthetase incorporated into an *Escherichia coli* (E. coli) vector, to a process for producing glutathione using said plasmid, and to cells of E. Coli transformed with such plasmids.

Glutathione is a tripeptide consisting of the amino acids L-glutamic acid L-cysteine and glycine, and is an important compound used as a therapeutic agent for the treatment of liver diseases, and as a biochemical reagent. Conventionally, glutathione is prepared by either organic synthesis or extraction from microbial (especially yeast) cells. However, neither method is completely satisfactory; the former is complex and involves many and prolonged reaction steps, and the latter achieves low intracellular content of glutathione in spite of complicated procedures. Therefore, the development of a more efficient process for glutathione production has been desired. The present inventors have combined biochemical techniques with DNA recombinant technology and have succeeded in producing glutathione by culturing a bacterium of E. coli that has been transformed to acquire the ability to synthesize glutathione.

Glutathione (hereunder referred to as GSH) is biosynthesized from L-glutamic acid, L-cysteine and glycine through reactions that are catalyzed by two enzymes, γ - glutamyl - L - cysteine synthetase (hereunder GSH-I) and glutathione synthetase (hereunder GSH-II), and which require adenosine - 5' - triphosphate (hereunder ATP). The present inventors previously introduced an enhanced activity of the first enzyme GSH-I into a bacterium of E. coli by recombinant-DNA technology and grew the strain to show its ability to produce GSH in high yields (EP 71486). As a result of further studies to improve this strain, the present inventors have succeeded in the cloning of a gene for the second GSH synthesizing enzyme GSH-II (this gene is hereunder referred to as gsh II) and in obtaining strains of E. coli with enhanced GSH-II activity. The present invention provides a hybrid plasmid with enhanced GSH-II activity, a hybrid plasmid with enhanced activities of both GSH-I and GSH-II, cells of E. coli transformed with such plasmids and an enzymatic process for producing glutathione using these plasmids.

The present invention is hereunder described in the order: cloning of a gene for GSH-II; cloning of a gene for enhancing the activities of both GSH-I and GSH-II; and processes for producing glutathione using these plasmids. The present invention may use any E. coli vector plasmid or phage having a relatively large copy number, and the following description assumes the use of pBR 322 and pBR 325 plasmids.

In the accompanying drawings:

Fig. 1(a) is a circular restriction map of recombinant DNA, pBR 322-gsh II, wherein ▨ indicates chromosomal DNA fragments of RC 912 and □ denotes pBR 322;

Fig. 1(b) is a similar map of another recombinant DNA according to the invention;

Fig. 2 is a circular restriction map of recombinant DNA, pBR 322-gsh I, wherein ■ indicates chromosomal DNA fragments of RC 912, and □ denotes pBR 322;

Fig. 3 shows the construction and circular restriction map of recombinant DNA, pBR 322-dsh I · II, wherein ▨ indicates chromosomal DNA fragments of RC 912 (gsh II), ■ denotes chromosomal DNA fragments of RC 912 (gsh I), and □ represents pBR 322; and

Fig. 4 shows the construction and circular restriction map of recombinant DNA, pBR 325-gsh I · II, wherein ▨ indicates chromosomal DNA fragments of RC 912 (gsh II), ■ denotes chromosomal DNA fragments of RC 912 (gsh I), and ▨ and □ represent pBR 322 and pBR 325 respectively.

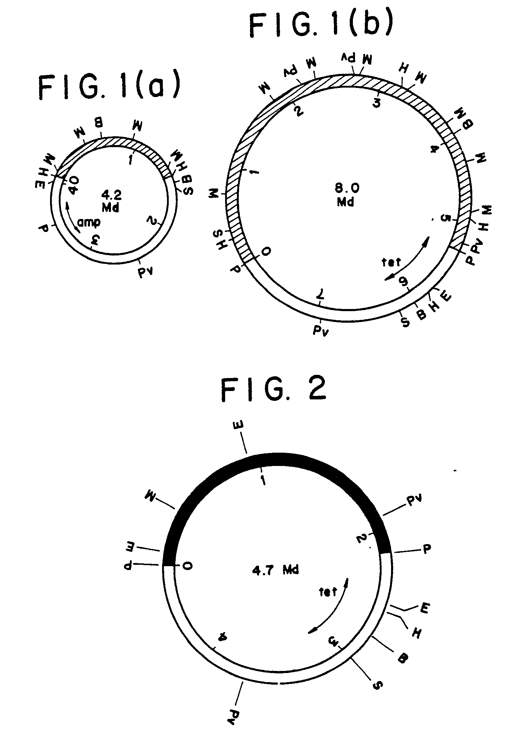

In Figs. 1 to 4, the following restriction enzymes are designated by the accompanying symbols in parentheses: PstI (P), EcoRI (E), BamHI (B), SalI (S), MluI (M), HindIII (H), and PvuII (Pv). The numerical figures in the respective rings represent the molecular weights of the plasmids in megadaltons.

Cloning of a gene for GSH-II

In the present invention, gsh II, the gene for GSH-II, can be cloned as follows. First, revertant mutant RC 912 of GSH-I deficient strain C 912 is induced by mutation from a parent bacterium E. coli B (ATCC 23226) according to the method described in Agric. Biol. Chem., 45(9), 2131 (1981). Then, chromosomal DNA is isolated from RC 912 by a known method, for example, the phenol extraction method (Biochim. Biophys. Acta, 72, 619—629 (1963), and is cut into fragments by a restriction endonuclease, say, HindIII; vector pBR 322 is digested with the same restriction endonuclease and treated with an alkali phosphatase according to the method described in Science, 196, 1313—1319 (1977).

The so treated pBR 322 is mixed with the previously obtained chromosomal DNA fragments, and the mixture is annealed at 75°C for 5 minutes and ligated with a $T_4$ DNA ligase to prepare a recombinant DNA. This process may use any kind of restriction endonuclease and requires no treatment with alkali phosphatase. The whole recombinant DNA is then introduced into the cells of GSH-II deficient strain C 1001 that has been derived from E. coli B by mutation and which has been rendered competent by treatment with calcium ions [Molec. gen. Genet., 124, 1—10 (1973)]. In order to select cells carrying the desired GSH-II gene (gsh II) from among the DNA-introduced cells, the latter are spread over a minimum agar plate containing 80 µg/ml of tetramethylthiuram disulfide (hereunder TMTD) and 5 µg/ml of ampicillin (hereunder AM) or tetracycline (hereunder Tc). The other components of this agar plate which is hereunder referred to as a DM medium are as follows: $KH_2PO_4$ (0.3%), $K_2HPO_4$ (0.7%), $MgSO_4 \cdot 7H_2O$ (0.01%), $(NH_4)_2SO_4$ (0.1%), glucose (0.5%) and agar (1.5%). After incubation at 37°C for 10—40 hours, large colonies are formed. By separating these colonies, the cells carrying the desired gene gsh II can be easily obtained.

The recombinant DNA present in this strain is designated as pBR 322-gsh II. The strain carrying this recombinant DNA is cultured on an L-medium (containing 0.5% yeast extract, 0.1% glucose, 0.5% NaCl and 1.0% peptone, pH: 7.2) well into the logarithmic growth phase. Then, 150 µg/ml of chloramphenicol (hereunder Cm) is added to the medium, and the incubation is continued for an additional 16 hours so as to increase the amount of the intracellular recombinant DNA. The microbial cells are collected and subjected to density gradient centrifugation by a conventional method [Nucleic Acid Res., 7, 1513—1517 (1979)] in order to obtain a large amount of pBR 322-gsh II. The resulting recombinant DNA: pBR 322-gsh II has a molecular weight of 4.2 megadaltons (4.2 Md) and has a 1.6 Md segment of RC 912-derived chromosomal DNA introduced in the HindIII site of pBR 322, as shown in Fig. 1(a). By treating the same RC 912-derived chromosomal DNA with PstI, a recombinant DNA of the type shown in Fig. 1(b) can also be prepared. A strain having the activity of GSH-II specifically enhanced can be obtained by introducing the recombinant DNA: pBR 322-gsh II into a mutant of E. coli, such as RC 912 or C 600. C 600 is described in "Gene" January 1980 p. 138 and is commercially available.

Cloning of a gene for enhanced activity of both GSH-I and GSH-II

Strains with simultaneous enhancement of the activities of both GSH-II and GSH-I can be produced by the following methods. A first method consists of incorporating both pBR 322-gsh II and pBR 322-gsh I into an E. coli derived mutant. As described in EP 71486, pBR 322-gsh I is the recombinant DNA prepared by treating RC 912 derived chromosomal DNA with the restriction endonuclease PstI. this recombinant DNA has a molecular weight of 4.7 Md and has a 2.1 Md segment of RC 912 chromosomal DNA inserted into the PstI site of pBR 322 (see Fig. 2).

The two recombinant DNAs are introduced into an E. coli mutant that has been rendered competent by the method described in Molec. gen. Genet., 124, 1—10 (1973). The order of their introduction is not important and if necessary, the two may be simultaneously introduced in the form of a mixture. Cells having these hybrid plasmids introduced thereinto may be selected for their resistance to TMTD or sensitivity to Am or Tc. For example, cells having pBR 322-gsh I introduced into the GSH-I activity deficient strain C 912 induced from E. coli B by mutation can be easily selected as colonies that grow on an L-medium containing 20 µg/ml of Tc. Those having pBR 322-gsh II, the plasmid of Fig. 1(a), introduced into C 912 can be selected as colonies growing on an L-medium containing 20 µg/ml of Am. If the DM medium as defined hereinbefore is used in place of the L-medium, the cells can be selected as colonies resistant to 10—20 µg/ml of TMTD. Cells having both pBR 322-gsh I and pBR 322-gsh II introduced into C 912 can be easily selected as colonies growing on an L-medium containing 20 µg/ml of Am and an equal amount of Tc.

By the procedure described above, a strain having the two recombinant DNAs, pBR 322-gsh I and pBR 322-gsh II, within the same cell and which has an enhanced activity of both GSH-I and GSH-II can be obtained.

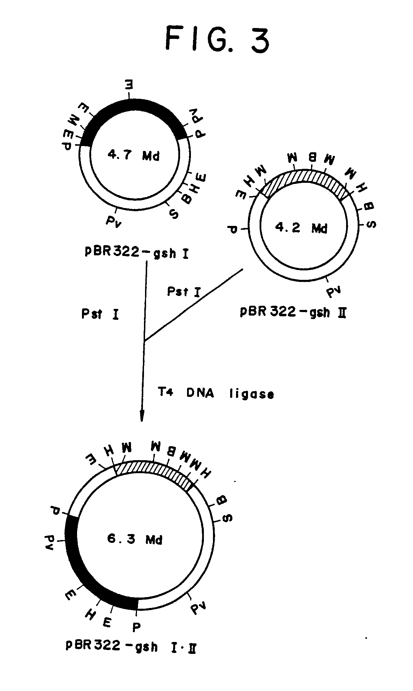

A second method of enhancing the activities of both GSH-I and GSH-II is by linking genes for the two enzymes with the same vector and introducing them ensemble into an E. coli strain. This method proceeds as follows. First, pBR 322-gsh I is digested with PstI, and the released RC 912 derived DNA fragments are isolated. This can be easily accomplished by separating the PstI digested pBR 322-gsh I by agarose gel electrophoresis and extracting the separated product from the gel [see "Methods in Enzymology", 68, 176—182 (1979)]. The isolated DNA fragments are mixed with the PstI treated pBR 322-gsh II, the plasmid of Fig. 1(a). The mixture is ligated with $T_4$ DNA ligase and introduced into an E. coli strain, such as C 912 strain, that has been rendered competent by treatment with calcium ions. Cells harboring a recombinant DNA carrying the desired gsh I and gsh II (said DNA is hereunder referred to as pBR 322-gsh I · II) can be easily selected by checking if the strain C 912 that has been subjected to transformation can grow on a DM-agar plate containing 10 µg/ml of TMTD (see Fig. 3).

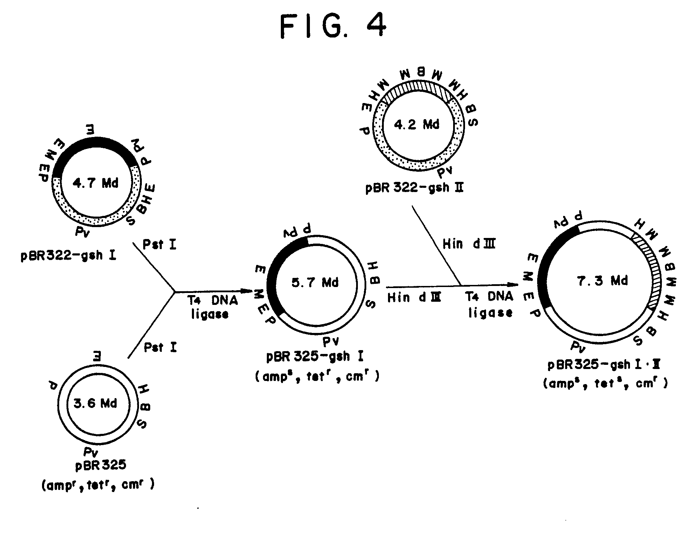

In either case, the recombinant DNA: pBR 322-gsh I · II carrying the two genes gsh I and gsh II on the vector pBR 322 can be obtained, and by introducing this recombinant DNA into an E. coli strain, the latter can be simultaneously provided with enhanced activities of GSH-I and GSH-II. In the above described method of enhancing the activities of GSH-I and GSH-II simultaneously, the vector pBR 322 may be replaced by pBR 325. The two genes for GSH-I and GSH-II (gsh-I and gsh-II) can be cloned onto the vector pBR 325 by the method illustrated in Fig. 4 which is essentially the same as that described for the case using pBR 322 as the vector plasmid. First, pBR 322-gsh I is digested with PstI to recover DNA fragments carrying gsh I. Likewise, pBR 322-gsh II is digested with HindIII and DNA fragments carrying gsh II are collected. The two types of DNA fragments are introduced into the PstI and HindIII sites of 325 so as to construct the recombinant DNA: pBR 325-gsh-I · II carrying both gsh I and gsh II on the vector pBR 325. This method has the advantage that the transformed cells can be selected very easily because the vector plasmid pBR 325 bears a gene for Cm resistance. By incorporating pBR 325-gsh I · II into an E. coli strain, the latter can be simultaneously provided with enhanced activities of GSH-I and GSH-II.

Production of glutathione

The bacterium of E. coli that has the enhanced activity of GSH-II or both GSH-I and GSH-II can be used in the production of GSH by the following procedure. First, the bacterium having the enhanced activity of GSH-II or both GSH-I and GSH-II is transferred to a nutrient medium containing a carbon source, a nitrogen source and an inorganic salt, or a minimum medium (e.g. DM medium) and is then cultured with shaking.

Examples of the carbon source include glucose, fructose, glycerin and sucrose. Examples of the nitrogen source include inorganic compounds such as ammonium chloride, ammonium nitrate, ammonium sulfate and urea, as well as organic compounds such as yeast extract, peptone and meat extract. Magnesium chloride and manganese chloride are effective as sources of trace metallic elements. The carbon sources are suitably used in a concentration of 0.1 to 5%, preferably 0.5%. The nitrogen sources are e.g. in a concentration of 0.05 to 5%, preferably 0.5%. The sources of trace metallic elements are preferably used in a concentration of about 0.005%. The culturing temperature e.g. from 20 to 40°C, and 37°C is preferred. The culturing is preferably effected at pH 6 to 8, e.g. at pH 7. After completion of the incubation, the microbial cells are collected from the culture liquor and washed once with 0.85% physiological saline to form an aqueous suspension, which is then immersed in boiling water (100°C) for 1 to 10 minutes, preferably for 1 minute, to thereby extract GSH from E. coli cells. Alternatively, the microbial cells collected from the culture liquor are given one of the treatments mentioned below and are then reacted with glutamic acid, cysteine, glycine, magnesium ions, and ATP, preferably in the presence of a suitable ATP regeneration system. Among the treatments of the microbial cells collected from the culture liquor are treatment with an organic solvent, treatment with a surfactant, ultrasonic homogenization, centrifugation after ultrasonic homogenization to obtain a cell-free extract, and immobilizing the microbial cells or enzymes with a suitable support. Examples of the organic solvent that can be used are acetone, toluene and ether. Examples of the surfactant that may be used include Triton X100, dodecyl sulfate, and cetyltrimethylammonium bromide. Suitable supports for immobilizing the microbial cells or enzymes include polyacrylamide gel, carrageenan gel, alginate gel, photo-crosslinkable resins, as well as DEAE-cellulose and DEAE-Sephadex (commercially available from Pharmacia Fine Chemicals AB, Uppsala, Sweden).

The ATP regeneration system that may be used in the reaction for the synthesis of GSH may advantageously use the reactions of acetate kinase and carbamate kinase present in E. coli or the glycolysis occurring in microorganisms. The reaction for the production of GSH may be effected by contacting the cells (treated) or the enzyme extract with a reaction solution containing 10—100 mM, preferably 80 mM, of L-glutamic acid, 5—40 mM, preferably 20 mM, of L-cysteine, 5—50 mM, preferably 20 mM, of glycine, 1—30 mM, preferably 10 mM, of magnesium ions and 1—20 mM, preferably 10 mM, of ATP. The contact time is several hours. The reaction temperature is e.g. from 20° to 40°C and 37°C is preferred. The pH for the reaction is e.g. from 6 to 9, with 7.5 being preferred. If the reaction of acetate kinase is used as the ATP regeneration system, 5 to 40 mM, preferably 20 mM, of acetyl Δ phosphate may additionally be used. No external source of acetate kinase need be added because the bacteria of E. coli are high in acetate kinase activity.

The GSH extracted or formed in the reaction solution by the procedure described above can be easily isolated by using a conventional column of ion exchange resin. First, the pH of the extract or the reaction solution is adjusted to 3.0 with sulfuric acid, and the so adjusted extract or reaction solution is passed through a cation exchange resin such as Diaion PK-228 $H^+$ (commercially available from Mitsubishi Chemical Industries Limited, Tokyo) to adsorb GSH onto the resin, from which GSH is eluted with 0.5 M $NH_4OH$. The eluate is adjusted to pH 4.5 with $H_2SO_4$ and passed through an anion exchange resin such as Duolite A2 (of $CH_3COO^-$ form, commercially available from Diamond Alkali Co., U.S.A.) to adsorb GSH onto the resin. The adsorbed GSH is eluted with 0.5 M sulfuric acid. By adding ethanol up to a content of 50%, GSH in crystalline form can be isolated from the eluate.

Strains wherein pBR 322-gsh II and pBR 325-gsh I · II are introduced into RC 912 have been deposited with the Fermentation Research Institute under the following respective deposit numbers: FERM BP-336 and FERM BP-337.

FERM

The present invention is illustrated by the following working Examples.

Example 1

Construction of pBR 322-gsh II and introduction of the same into E. coli mutants

Mutant RC 912 induced from E. coli B (ATCC 23226) was grown on an L-medium well into the logarithmic phase, and the microbial cells were collected and washed once with physiological saline. About 1.6 mg of chromosomal DNA was isolated from 1 g (wet weight) of the microbial cells by the phenol extraction method described in Biochim. Biophys. Acta., 72, 619—629 (1963). A 1 μg portion of this chromosomal DNA was cleaved into fragments by digestion with HindIII at 37°C for 30 minutes. The fragments were mixed with 1 μg of vector plasmid pBR 322 that had been prepared by a 2-hr digestion with HindIII and subsequent treatment with alkali phosphatase according to the method of Ullrich et al. described in Science, 196, 1313—1319 (1977), and the mixture was ligated with $T_4$ DNA ligase at 10°C for 16 hours to construct a recombinant DNA.

The whole recombinant DNA was introduced into competent cells of GSH-II deficient strain C 1001 that had induced by mutation from E. coli B, and the cells were spread on a DM medium containing 80 μg/ml of TMTD. After incubation at 37°C for 40 hours, the resulting large colonies were collected. The colonies collected contained a recombinant DNA: pBR 322-gsh II. A large amount of the desired pBR 322-gsh II was isolated from one of the colonies by density gradient centrifugation. The constructed recombinant DNA had the recognition sequences for restriction endonuclease shown in Fig. 1(a). The DNA had a molecular weight of 4.2 Md and had a 1.6 Md segment of RC 912 derived chromosomal DNA incorporated in the HindIII site of vector pBR 322. A similar recombinant DNA could be constructed by using PstI in place of HindIII. This DNA had the recognition sequences for restriction endonuclease shown in Fig. 1(b). It had a molecular weight of 8.0 Md and had a 5.4 Md segment of RC 912 derived chromosomal DNA incorporated in the PstI site of pBR 322.

The recombinant DNA: pBR 322-gsh II shown in Fig. 1(a) was introduced into the E. coli B mutants listed in Table 1. Before the introduction of the recombinant DNA, competent cells were produced by treating the mutants with calcium ions according to the method described in Molec. gen. Genet., 124, 1—10 (1973). The transformed strains were selected by checking if the microbial cells grew on an L-medium containing 20 µg/ml of Am or a DM medium containing 10—80 µg/ml of TMTD. The activities of GSH-I and GSH-II present in the transformed cells are listed in Table 1. The activities of the respective enzymes were measured by the method described in J. Gen. Microbiol., 128, 1047—1052 (1982) using extracts prepared from microbial cells cultured on a DM medium until the logarithmic growth phase.

TABLE 1
GSH-I and GSH-II activities of cells carrying recombinant DNA:
pBR 322-gsh II

| Strain | Enzyme activity (µmole/hr/mg-protein) | | | |
|---|---|---|---|---|
| | GSH-I | | GSH-II | |
| C912 | 0 | (—) | 0.60 | (1.0) |
| C912/pBR 322-gsh II | 0 | (—) | 22.0 | (36.7) |
| C1001 | 0.06 | (1.0) | 0 | |
| C1001/pBR 322-gsh II | 0.05 | (1.0) | 20.2 | |
| RC912 | 0.06 | (1.0) | 0.57 | (1.0) |
| RC912/pBR 322-gsh II | 0.06 | (1.0) | 18.8 | (31.2) |

N.B. The figures in parentheses are relative values.

Example 2

The strains listed in Table 1 of Example 1 were transferred to 500 ml each of DM media and cultured at 37°C for 3 hours with shaking until the logarithm growth phase. The microbial cells were collected and washed once with 0.85% physiological saline. The cells were resuspended in water to a concentration of 50 mg/ml. The suspension (0.5 ml) was heated at 100°C for 1 minute and glutathione was extracted from the cells. The glutathione contents for the respective strains were determined by the method described in Anal. Biochem., 27, 502—522 (1969) and the results are listed in Table 2.

TABLE 2
Glutathione contents of cells carrying recombinant DNA:
pBR 322-gsh II

| Strain | Glutathione content (mg/g-wet cells) |
|---|---|
| C912 | 0 |
| C912/pBR 322-gsh II | 0 |
| C1001 | 0 |
| C1001/pBR 322-gsh II | 1.2 |
| RC912 | 0.7 |
| RC912/pBR 322-gsh II | 1.4 |

Example 3

Construction of strains carrying pBR 322-gsh I and pBR 322-gsh II

Strains C 912/pBR 322-gsh II, C 1001/pBR 322-gsh II and RC 912/pBR 322-gsh II were rendered competent by treatment with calcium ions. The recombinant DNA: pBR 322-gsh I was introduced into the cells of the respective strains so as to transform them to cells each containing both pBR 322-gsh I and pBR 322-gsh II. The activities of GSH-I and GSH-II in the transformed cells and the contents of intracellular glutathione are shown in Table 3.

TABLE 3
GSH-I and GSH-II activities and GSH contents of
cells carrying both pBR 322-gsh I and pBR 322-gsh II

| Strain | Enzyme activity (μmole/hr/mg-protein) | | | | Glutathione content (mg/g-wet cells) | |
|---|---|---|---|---|---|---|
| | GSH-I | | GSH-II | | | |
| C912 | 0 | | 0.6 | (1.0) | 0 | |
| C912/pBR 322-gsh I · -gsh II | 1.5 | | 9.1 | (15.1) | 1.3 | |
| C1001 | 0.06 | (1.0) | 0 | | 0 | |
| C1001/pBR 322-gsh I · -gsh II | 1.7 | (28.3) | 10.0 | | 1.5 | |
| RC912 | 0.06 | (1.0) | 0.6 | (1.0) | 0.6 | (1.0) |
| RC912/pBR 322-gsh I · -gsh II | 1.7 | (28.3) | 10.1 | (16.8) | 1.7 | (2.8) |

N.B. The figures in parentheses are relative values.

Example 4
Construction of pBR 322-gsh I · II and introduction of the same into E. coli mutants

Recombinant DNA pBR 322-gsh I (50 μg) was digested with PstI and the resulting DNA fragments were separated by agarose gel electrophoresis. The gel containing DNA fragments was cut out, placed in a dialyzing tube and again subjected to electrophoresis so as to extract DNA fragments from the gel. The extracted DNA fragments contained about 5 μg of gsh I.

The other recombinant DNA pBR 322-gsh II (1 μg) was digested with PstI and mixed with 1 μg of the previously obtained DNA fragments containing gsh I. The mixture was ligated with $T_4$ DNA ligase. As a result, a recombinant DNA: pBR 322-gsh I · II carrying both gsh-I and gsh-II on vector plasmid pBR 322 was constructed. This recombinant DNA was introduced into cells of the E. coli B mutants indicated in Table 4 that had been rendered competent by treatment with calcium ions. The transformed cells could be easily selected by collecting the large colonies growing on a DM medium containing 20 μg/ml of TMTD. The activities of GSH-I and GSH-II in the transformed cells and the contents of intracellular glutathione are shown in Table 4.

TABLE 4
GSH-I and GSH-II activities and GSH contents of
cells carrying pBR 322-gsh I · II

| Strain | Enzyme activity (μmole/hr/mg-protein) | | | | Glutathione content (mg/g-wet cells) | |
|---|---|---|---|---|---|---|
| | GSH-I | | GSH-II | | | |
| C912 | 0 | | 0.60 | (1.0) | 0 | |
| C912/pBR 322-gsh I · II | 1.5 | | 12.0 | (2.0) | 1.6 | |
| C1001 | 0.06 | (1.0) | 0 | | 0 | |
| C1001/pBR 322-gsh I · II | 1.60 | (26.7) | 11.80 | | 1.5 | |
| RC912 | 0.06 | (1.0) | 0.57 | (1.0) | 0.5 | (1.0) |
| RC912/pBR 322-gsh I · II | 1.74 | (29.0) | 15.8 | (27.7) | 1.7 | (3.4) |

N.B. The figures in parentheses are relative values.

Example 5
Construction of pBR 325-gsh I · II and introduction of the same into E. coli mutants

Recombinant DNA pBR 322-gsh I (50 μg) was digested with PstI and subsequently treated as in Example 4 to obtain 4 μg of DNA fragments containing gsh I. Vector plasmid pBR 325 was digested with PstI and mixed with 1 μg of the previously obtained DNA fragments containing gsh I. The mixture was

ligated with $T_4$ DNA ligase. The so treated mixture was introduced into the cells of strain C 912, and the transformed cells carrying the desired recombinant DNA pBR 325-gsh I were obtained by selecting the colonies growing on a DM medium containing 20 µg/ml of TMTD and 5 µg/ml of Tc. A large amount of pBR 325-gsh I was isolated from one of the colonies by density gradient centrifugation.

The other recombinant DNA pBR 322-gsh II (50 µg) was digested with HindIII, and as in Example 4, the resulting DNA fragments were separated by electrophoresis and subsequently treated to obtain about 7 µg of DNA fragments containing gsh II. A 1 µg portion of these DNA fragments was mixed with 1 µg of the previously obtained pBR 325-gsh I after digestion with HindIII. The mixture was ligated with $T_4$ DNA ligase. As a result, a recombinant DNA: pBR 325-gsh I · II carrying both gsh-I and gsh-II on vector pBR 325 could be constructed *in vitro*. This recombinant DNA was introduced into cells of the E. coli B mutants listed in Table 5 that had been rendered competent by treatment with calcium ions. The transformed cells could be selected by collecting the large colonies growing on a DM medium containing 80 µg/ml of TMTD and 2 µg/ml of Cm. The activities of GSH-I and GSH-II in the transformed cells and the contents of intracellular glutathione are shown in Table 5.

TABLE 5
GSH-I and GSH-II activities and GSH contents
of cells carrying pBR 325-gsh I · II

| Strain | Enzyme activity (µmole/hr/mg-protein) | | | | Glutathione content (mg/g-wet cells) | |
|---|---|---|---|---|---|---|
| | GSH-I | | GSH-II | | | |
| C912 | 0 | | 0.60 | (1.0) | 0 | |
| C912/pBR 325-gsh I · II | 1.4 | | 11.2 | (18.7) | 1.7 | |
| C1001 | 0.06 | (1.0) | 0 | | 0 | |
| C1001/pBR 325-gsh I · II | 1.57 | (26.2) | 10.8 | | 1.9 | |
| RC912 | 0.06 | (1.0) | 0.57 | (1.0) | 0.5 | (1.0) |
| RC912/pBR 325-gsh I · II | 1.65 | (27.5) | 14.60 | (25.6) | 2.3 | (4.6) |

N.B. The figures in parentheses are relative values.

Example 6

Strains RC 912/pBR/322-gsh II (Table 2 of Example 2), RC 912/pBR 322-gsh I and RC 912 pBR 322-gsh II (Table 3 of Example 3), RC 912/pBR 322-gsh I, -gsh II (Table 4 of Example 4) and RC 912/pBR 325-gsh I · II (Table 5 of Example 5) were cultured on DM media as in Example 2. The microbial cells in the logarithmic growth phase were collected, washed once with 0.85% physiological saline, suspended in 5 mM tris-HCl buffer solution (pH: 7.5), homogenized by ultrasonic treatment (90 kHz/5 min) and centrifuged at 15,000 rpm for 30 minutes. The resulting cell extract was incubated at 37°C for 2 hours in a reaction solution containing 20 mM L-glutamic acid, 20 mM L-cysteine, 20 mM glycine, 10 mM magnesium chloride, 10 mM ATP, 10 mM acetylphosphate and 50 mM tris-HCl buffer solution (pH: 7.0). The amounts of glutathione produced in the reaction liquors for the respective strains are listed in Table 6.

TABLE 6
Glutathione production of RC 912
strains with various recombinant DNAs

| Strain | GSH producing activity (mg/ml/2 hr) |
|---|---|
| RC912 | 0.4 |
| RC912/pBR 322-gsh II (FERM BP-336) | 0.8 |
| RC912/pBR 322-gsh I · -gsh II | 2.1 |
| RC912/pBR 322-gsh I · II | 2.3 |
| RC912/pBR 325-gsh I · II (FERM BP-337) | 2.8 |

Example 7

Strain RC 912/pBR 325-gsh I · II (Table 5 of Example 5) was transferred to 1000 ml of a medium (pH: 7.0) containing 1.0% peptone, 1.0% yeast extract, 0.5% meat extract, 1.0% glucose, 0.01%

8

MgSO$_4$ · 7H$_2$O, 0.5% KH$_2$PO$_4$ and 20 µg/ml of Cm, and cultured at 30°C for 20 hours under shaking. The microbial cells were collected and washed once with physiological saline. The harvested cells (28 g on a wet basis) were suspended in 15 ml of 30% aqueous potassium chloride. To the suspension, 13 ml of 33.5% acrylamide monomer, 2 ml of 20% N,N' - methylenebisacrylamide, 5 ml of 5.0% 3 - dimethylaminopropionitrile and 6 ml of 6.5% potassium persulfate were added, and the mixture was left to stand at 20°C until it gelled. The immobilized cells were cut into cubes 2 mm long on each side and washed with physiological saline to provide 80 g of immobilized E. coli RC 912/pBR 325-gsh I · II. Cells of plasmid-free RC 912 were likewise immobilized.

Each group of the immobilized cells (25 g) was put into 5 ml of a reaction solution containing 80 mM L-glutamic acid, 20 mM L-cysteine, 20 mM glycine, 25 mM magnesium chloride, 20 mM ATP, 20 mM acetyl Δ phosphate, and 25 mM potassium phosphate buffered solution (pH: 7.0) and subjected to reaction at 37°C with shaking. The amounts of glutathione formed in the reaction solution were determined after the 1st hour, 2nd hour and 4th hour of the reaction. The results are shown in Table 7, wherein the "percent conversion" indicates the conversion to GSH from L-cysteine.

TABLE 7

| Reaction time | RC 912 | | RC 912/pBR 325-gsh I · II | |
|---|---|---|---|---|
| (hr) | GSH conc. (mM) | Percent conversion | GSH conc. (mM) | Percent conversion |
| 1 | 0.8 | 4 | 13.5 | 67 |
| 2 | 1.0 | 5 | 16.6 | 83 |
| 4 | 2.4 | 12 | 18.2 | 91 |

Example 8

Strains RC 912/pBR 325-gsh I · II and RC 912 (Table 5 of Example 5) were cultured on the same medium as used in Example 7. 10 g (wet) of collected microbial cells were washed once with physiological saline, suspended in 10 ml of physiological saline and heated at 37°C. The heated suspension was mixed with 20 ml of 3.1% aqueous carrageenan (37°C). The mixture was dropped into 2% aqueous potassium chloride through a nozzle to produce gel beds having a diameter of about 3 mm. The immobilized cells (2.5 g) were put into 5 ml of a reaction liquor having the same compositions as used in Example 7, and subjected to reaction at 37°C with shaking. The amounts of glutathione formed in the reaction solution were determined after the 1st, 2nd and 4th hours of the reaction. The results are shown in Table 8, wherein the "percent conversion" indicates the conversion to GSH from L-cysteine. After completion of the 4th hour of reaction, the immobilized cells were filtered and subjected to repeated reaction in reaction solution having the same composition as defined in Example 7. The amounts of glutathione formed after 4 hrs in repeated reactions were determined, and the results are shown in Table 9. Similar results were obtained even when the concentrations of ATP and acetylphosphate in reaction solution were varied.

TABLE 8

| Reaction time | RC 912 | | RC 912/pBR 325-gsh I · II | |
|---|---|---|---|---|
| (hr) | GSH conc. (mM) | Percent conversion | GSH conc. (mM) | Percent conversion |
| 1 | 1.0 | 5 | 13.6 | 68 |
| 2 | 1.9 | 10 | 18.4 | 92 |
| 4 | 2.3 | 12 | 18.6 | 93 |

# EP 0 107 400 B1

TABLE 9

| No. of repetitions of reaction | RC 912 | | RC 912/pBR 325-gsh I · II | |
|---|---|---|---|---|
| | GSH conc. (mM) | Percent conversion | GSH conc. (mM) | Percent conversion |
| 1 | 2.3 | 12 | 18.6 | 93 |
| 5 | 2.0 | 10 | 17.4 | 87 |
| 10 | 1.8 | 9 | 17.4 | 87 |

## Claims

1. A hybrid plasmid which can be replicated by a bacterium of the species *Escherichia coli* and has incorporated into the DNA of an *Escherichia coli* vector a gene for glutathione synthetase (GSH II), which gene can be isolated from revertant mutant *E. coli* RC 912 by treatment of chromosomal DNA thereof with restriction enzyme PstI or HindIII.

2. A hybrid plasmid according to claim 1 wherein the vector also incorporates a gene for γ - glutamyl - L - cysteine synthetase (GSH I) which is obtainable from revertant mutant *E. coli* RC 912 by treatment of chromsomal DNA thereof with restriction enzyme PstI.

3. A hybrid plasmid according to claim 1 or 2 in which the vector is plasmid pBR 322.

4. A hybrid plasmid according to claim 1 which is vector plasmid pBR 322 carrying a gene for coding GSH II (hybrid pBR 322-gsh II deposited as FERM BP-336).

5. A hybrid plasmid according to claim 2 which is vector plasmid pBR 325 carrying genes for coding GSH I and GSH II (hybrid pBR 325-gsh I · II deposited as FERM BP-337).

6. Cells of *Escherichia coli* transformed with a hybrid plasmid according to any one of claims 1 to 5, or cells of *Escherichia coli* transformed with a first plasmid according to claim 1 and with a different second hybrid plasmid which has incorporated in an *Escherichia coli* vector a gene for γ - glutamyl - L - cysteine synthetase without a gene for glutathione synthetase, which incorporated gene is obtainable from revertant mutant *E. coli* RC 912 by treatment of chromosomal DNA thereof with restriction enzyme PstI.

7. A process for producing glutathione which comprises culturing cells according to claim 6 and then recovering glutathione from the culture broth or reacting the cultured cells or an active extract therefrom with a reaction mixture comprising L-glutamic acid, L-cysteine, glycine, adenosine - 5' - triphosphate and magnesium ions to form glutathione.

8. A process according to claim 7 wherein the reactant mixture includes an adenosine - 5' - triphosphate regeneration system, e.g. one using the reaction of acetate kinase, the reaction of carbamate kinase or the glycolytic reaction of bacteria or yeasts.

9. A process according to claim 7 or 8 wherein the reactant mixture is reacted with a cell-free extract from the cultured cells, or a suspension of the cultured cells, or the immobilized cultured cells or immobilized enzyme therefrom.

10. A process according to claim 9 wherein the reactant mixture is reacted with immobilized cultured cells fixed on a gel support, e.g. of polyacrylamide gel.

## Patentansprüche

1. Hybridplasmid, das durch ein Bakterium der Species Escherichia coli repliziert werden kann und das in die DNS eines Escherichia coli-Vektors ein Gen für Glutathionsynthetase (GSH II) inkorporiert hat, wobei das Gen aus revertierter Mutante E. coli RC 912 isoliert werden kann durch Behandlung deren Chromosomen · DNS mit Restriktionsenzym PstI oder HindIII.

2. Hybridplasmid nach Anspruch 1, wobei der Vektor auch ein Gen für γ - Glytamyl - L - cystein - Synthetase (GSH I) inkorporiert, das erhältlich ist aus revertierter Mutante E. coli RC 912 durch Behandlung deren Chromosomen · DNA mit Restriktionsenzym PstI.

3. Hybridplasmid nach Anspruch 1 oder 2, bei dem der Vektor Plasmid pBR 322 ist.

4. Hybridplasmid nach Anspruch 1, wobei der Vektor Plasmid pBR 322 ist, das ein Gen zur Codierung von GSH II trägt (Hybrid pBR 322-gsh II, hinterlegt als FERM BP-336).

5. Hybridplasmid nach Anspruch 2, wobei der Vektor Plasmid pBR 325 ist, das Gene zur Codierung von GSH I und GSH II trägt (Hybrid pBR 325-gsh I · II, hinterlegt als FERM BP-337).

6. Zellen von Escherichia coli, die transformiert sind mit einem Hybridplasmid nach einem der Ansprüche 1 bis 5, oder Zellen von Escherichia coli, die transformiert sind mit einem ersten Plasmid nach Anspruch 1 und mit einem unterschiedlichen zweiten Hybridplasmid, das in einen Escherichia coli-Vektor ein Gen für γ - Glutamyl - L - cystein - Synthetase ohne ein Gen für Glutathionsynthetase inkorporiert hat, wobei das inkorporierte Gen erhältlich ist aus revertierter Mutante E. coli RC 912 durch Behandlung deren Chromosomen-DNS mit Restriktionsenzym Pstl.

7. Verfahren zur Herstellung von Glutathion, umfassend die Züchtung von Zellen nach Anspruch 6 und anschließende Gewinnung von Glutathion aus der Kulturbrühe oder Umsetzung der gezüchteten Zellen oder eines aktiven Extraktes davon mit einem Reagensgemisch, umfassend L-Glutaminsäure, L-Cystein, Glycin, Adenosin - 5' - triphosphat und Magnesiumionen, zur Bildung von Glutathion.

8. Verfahren nach Anspruch 7, wobei das Reagensgemisch ein Adenosin - 5' - triphosphat - Regenerationssystem umfaßt, z.B. ein solches unter Anwendung der Reaktion von Acetatkinase, der Reaktion von Carbamatkinase oder der glykolytischen Reaktion von Bakterien oder Hefen.

9. Verfahren nach Anspruch 7 oder 8, wobei das Reagensgemisch umgesetzt wird mit einem zellfreien Extrakt von den gezüchteten Zellen oder einer Suspension der gezüchteten Zellen oder den immobilisierten gezüchteten Zellen oder immobilisiertem Enzym davon.

10. Verfahren nach Anspruch 9, wobei das Reagensgemisch umgesetzt wird mit immobilisierten gezüchteten Zellen, die auf einem Gelträger, z.B. Polyacrylamidgel, fixiert sind.

## Revendications

1. Plasmide hybride qui peut être répliqué par une bactérie de l'espèce *Escherichia coli* et a incorporé dans l'ADN d'un vecteur d'*Escherichia coli* un gène pour la glutathion-synthétase (GSH II), ledit gène pouvant être isolé à partir du mutant revertant *E. coli* RC 912 par traitement de son ADN chromosomique avec l'enzyme de restriction Pstl ou HindlII.

2. Plasmide hybride selon la revendication 1, dans lequel le vecteur incorpore également un gène pour la γ - glutamyl - L - cystéine synthétase (GSH I), que l'on peut obtenir à partir du mutant revertant *E. coli* RC 912 par traitement de son ADN chromosomique avec l'enzyme de restriction Pstl.

3. Plasmide hybride selon la revendication 1 ou 2, dans lequel le vecteur est le plasmide pBR 322.

4. Plasmide hybride selon la revendication 1, qui est le plasmide vecteur pBR 322 portant un gène codant pour GSH II (hybride pBR 322-gsh II déposé sous le n° FERM BP-336).

5. Plasmide hybride selon la revendication 2, qui est le plasmide vecteur pBR 325 portant des gènes codant pour GSH I et GSH II (hybride pBR 325-gsh I · II déposé sous le n° FERM BP-337).

6. Cellules d'*Escherichia coli* transformées avec un plasmide hybride tel que défini à l'une des revendications 1 à 5, ou cellules d'*Escherichia coli* transformées avec un premier plasmide tel que défini à la revendication 1 et avec un second plasmide hybride différente qui a incorporé dans un vecteur d'*Escherichia coli* un gène pour la γ - glutamyl - L - cystéine synthétase sans gène pour la glutathion-synthétase, ledit gène incorporé pouvant être obtenu à partir du mutant revertant *E. coli* RC 912 par traitement de son ADN chromsomique avec l'enzyme de restriction Pstl.

7. Procédé pour la production de glutathion, qui consiste à cultiver des cellules telles que définies à la revendication 6, puis à récupérer le glutathion à partir du bouillon de culture ou à faire réagir les cellules cultivées ou un extrait actif de celles-ci avec un mélange réactif comprenant l'acide L-glutamique, la L-cystéine, la glycine, l'adénosine - 5' - triphosphate et les ions magnésium, pour former le glutathion.

8. Procédé selon la revendication 7, dans lequel le mélange réactif comprend un système de régénération d'adénosine - 5' - triphosphate, par exemple celui utilisant la réaction de l'acétate kinase, la réaction de la carbamate kinase ou la réaction glycolytique de bactéries ou de levures.

9. Procédé selon la revendication 7 ou 8, dans lequel on fait réagir le mélange réactif avec un extrait exempt de cellules provenant des cellules cultivées, ou une suspension des cellules cultivées, ou les cellules cultivées immobilisées ou l'enzyme immobilisée à partir de celles-ci.

10. Procédé selon la revendication 9, dans lequel on fait réagir le mélange réactif avec des cellules cultivées immobilisées fixées sur un support de gel, par exemple, de gel de polyacrylamide.

EP 0 107 400 B1

FIG. 1(b)

FIG. 1(a)

FIG. 2

# FIG. 3

pBR322-gsh I

pBR322-gsh II

Pst I

Pst I

T4 DNA ligase

pBR322-gsh I·II

# FIG. 4